# EUROPEAN PATENT SPECIFICATION

(11) **EP 1 327 428 B1**
(45) Date of publication and mention of the grant of the patent: **17.07.2013**
(21) Application number: 02380269.7
(22) Date of filing: 18.12.2002
(51) Int. Cl.: A61F 13/476, A61F 13/15

(54) **Sanitary napkin or panty liner for use with a tanga undergarment**
Damenbinde oder Slipeinlage für Tanga-Slip
Serviette hygiénique ou protège-slip pour slip tanga

(30) Priority: 19.12.2001 MX PA02000071
(43) Date of publication of application: 16.07.2003
(73) Proprietor: Grupo P.I. Mabe, S.A. de C.V., 72230 Puebla, Estado de Puebla (MX)
(72) Inventor: Corona Carlos, Alberto, 72230 Puebla, Estado de Puebla (MX); Canales Espinosa De Los Monteros, Carlos, 72230 Puebla, Estado de Puebla (MX); Sanchez Fernandez, Lucia, 72230 Puebla, Estado de Puebla (MX); Rojas Oropeza, Lucia, 72230 Puebla, Estado de Puebla (MX)
(74) Representative: Gil-Vega, Victor

(56) References cited:
- EP-A- 1 138 295
- WO-A-00/72790
- DE-U- 20 002 192
- DE-U- 20 114 825
- US-A- 4 917 697
- US-A- 5 037 417
- US-A- 5 300 054
- US-A- 5 304 161
- US-A- 5 558 657
- US-A- 5 713 886
- US-A- 5 873 869
- US-A1- 2001 031 955
- US-B1- 6 443 934

## Description

### Background of the Invention

A sanitary napkin is a disposable absorbent article used by women during menses to protect their underwear. According, the sanitary napkin absorbs and retains menstrual fluids, preventing them from reaching the underwear of the user. On the other hand, a panty-liner is a disposable absorbent article used by women to protect their underwear from normal flows during the days menses is not present or during the final days of a menstrual period.

Articles of this type must be very comfortable, discreet and soft, and provide the desired protection; they must adapt to the central portion of the underwear, covering the zone where discharges will occur and, preferably, cover the lateral portions of the underwear to provide for better protection. In addition, they need to be capable of affixing to the underwear in a suitable manner, so that the user feels comfortable and safe at all times. There are in the market many types of sanitary napkins and panty-liners, designed for standard type and size underwear; however, when the user wears tanga-style underwear, she faces the problem resulting from the fact the sanitary napkin or the panty-liner is wider than the crotch portion of the underwear and projects outside same, making its use very uncomfortable.

There have been some efforts to provide users with a type of article to be used with tanga-style underwear, such as the one disclosed by U.S. Patent No. 5,683,373, to Kamela J. Darby, which discloses a V-shaped article with an upper portion in the shape of a bulb and a lower portion with a constant width of less than 25% of the width of the upper portion, so that it conforms to tanga-type underwear.

International publication WO 00/30585, SCA Hygiene Products, describes an article to be used with tanga-type underwear, having lateral edges which are essentially curved and the back section of which measures less than 4 cm. On the other hand, U.S. Patent No. 5,729,835, to Magda Williams, describes a panty-liner for use with tanga-style underwear including a flexible body and an enlarged configuration, wherein the width of the front section is higher than the width of the back section; the intermediate section decreases in width as it progresses towards the back section, so that the body of the panty-liner is formed by six superposed layers, one on top of the other.

None of the above-mentioned patents and publications focuses on the problem of the proper securing of the absorbent article to the underwear of the user, since although it is mentioned in all of said references that the article has an adhesive on the back portion in order to secure same to the underwear, this may not be sufficient, particularly since the back portion of same appears to be very narrow in order to achieve a good fastening.

The document US 5558657 by Hammons et al. is related to a disposable absorbent article, as a sanitary napkin, with wings or lateral projections divided in sections which can be folded in an independent way. These sections are overlapped one to another in the closest part of the absorbent core. When these wings are folded, there is interference of one section to another and they cover completely the central part of the disposable article. The wings disclosed in this document are not adequate to a disposable type tanga absorbent article. These wings of US 5558657 use an excessive wing material because the sections of the wings overlapped one to another. This excessive material is necessary because of the curvature of the central part of the absorbent core.

The documents US 5300054 and US 5304161, by Procter and Gamble, are related to a disposable sanitary napkin wherein the absorbent core has got several layers to sure a better absorption and retention. In these cases, the absorbent core is widest in the frontal section and it has not the adequate form to be used with a tanga type undergarment.

U.S. Patent No. 5,713,886, to David P. Sturino, discloses a sanitary napkin to be used with tanga-type underwear, which has two sections, a lower section with a constant width, and an upper section whose width increases up to the upper edge of the napkin, which also has wings or lateral projections extending from the longitudinal edges of the upper section thereof and a sole wing extending from one of the longitudinal edges of the lower section of the napkin. This design has the disadvantage that, since it has a sole wing by the lower section of the article, the same can be lifted by the wingless section, and further, the wingless section does not protect the edge of the underwear.

The present invention provides a disposable absorbent article, such as a sanitary napkin or a panty-liner, specifically designed to be used with tanga-type underwear, having wings or lateral projections along the entire length thereof, so to protect in an integral manner the edges of the tanga-type underwear, besides the fact that it achieves an adequate fastening of the article to the underwear, thus resulting in a very comfortable and safe article.

### Summary of the Invention

An object of the present invention is to provide a disposable absorbent article, such as a sanitary napkin or panty-liner, specifically designed to be used with tanga-type underwear, with wings or lateral projections which provide for better fastening and protection to the user.

A further object of the present invention is that wings or lateral projections provide an area of protection along the entire length of the article.

Another object of the present invention is that the article fastens adequately to the underwear, preventing same from lifting or narrowing as a result of the motions of the user.

### Brief Description of the Drawings

Figure 1 shows an embodiment of the article of the present invention.
Figure 2 shows an alternative of the article of the present invention, with wings of lateral projections which are narrower than those shown in Figure 1.
In Figures 3a, 3b and 3c the article of the invention is shown with wings or lateral projections and with different alternatives of absorbent cores.
Figure 4 shows an alternative of the invention, wherein the wings or lateral projections only span two thirds of the length of the absorbent core.
Figures 5 and 6 show alternatives for the wings or lateral projections of the article of the present invention.
Figure 7 is a section along line A-A' of Figure 1.
Figure 8 shows the crotch portion of tanga-type underwear along the part thereof where the wings or lateral projections will be affixed.

### Detailed Description of the Invention

There are many types of disposable absorbent articles, some of which are directed to babies and infants, such as disposable diapers for babies and training pants, among many others. Others are directed to feminine hygiene, such as sanitary napkins and panty-liners.

A sanitary napkin is a disposable absorbent article used by women during menses to contain and retain the menstrual fluids; a panty-liner is also a disposable absorbent article, worn by women during days when there is no menses or during the last days of the menstrual period, so to protect the underwear of the user from the normal fluids of a woman. There are in the market a great variety of designs of both sanitary napkins and panty-liners, adequate for the preferences of users.

Many women use as underwear tanga-type underwear, which narrows in the crotch section and remains narrow in the back portion thereof. Attempts to conform sanitary napkin and panty-liner designs to this type of underwear have begun. The present invention relates to a sanitary napkin or panty-liner adequate to be used with tanga-type underwear and also having wings or lateral projections to assist in the fastening thereof to the underwear and protect the longitudinal edges thereof in the crotch area.
Hereinafter, the invention will be described with reference to a sanitary napkin; however, it should be understood that same is true for a sanitary napkin, a panty-liner, or another catamenial device.

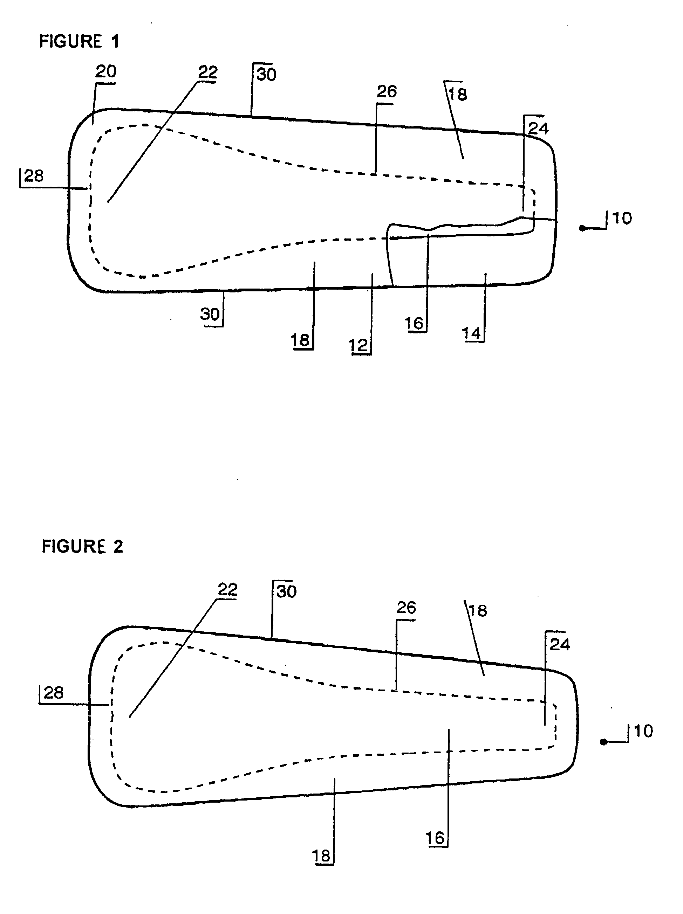

Figure 1 shows an alternative of the sanitary napkin (10) of the present invention, which consists of an upper layer (12), a lower layer (14), an absorbent core (16) and wings or lateral projections (18).

Upper layer (12) is the one that will be in contact with the body of the user and is a film permitting passage of fluids and preferably prevents their return. It must be soft and non irritant for the skin of the user; some of the films that have been used for this purpose are: non woven fabrics of polyester, polypropylene or mixtures thereof, micro-perforated polyethylene, laminates of micro-perforated polyethylene and non woven fabrics, and the like.

Lower layer (14) is the one that will be in contact with the underwear worn by the user and is a soft, thin and flexible film; it must be impervious or at least hydrophobic and preferably breathable. Some suitable materials for this purpose are low density polyethylene, a laminate of polyethylene with non woven fabric, hydrophobic non woven fabric, monolithic polyethylene, micro-perforated polyethylene, o any type of material meeting said characteristics. The lower layer (14) hast two faces, an inner face and an outer face, the latter to be in contact with the underwear of the user during use of the article.

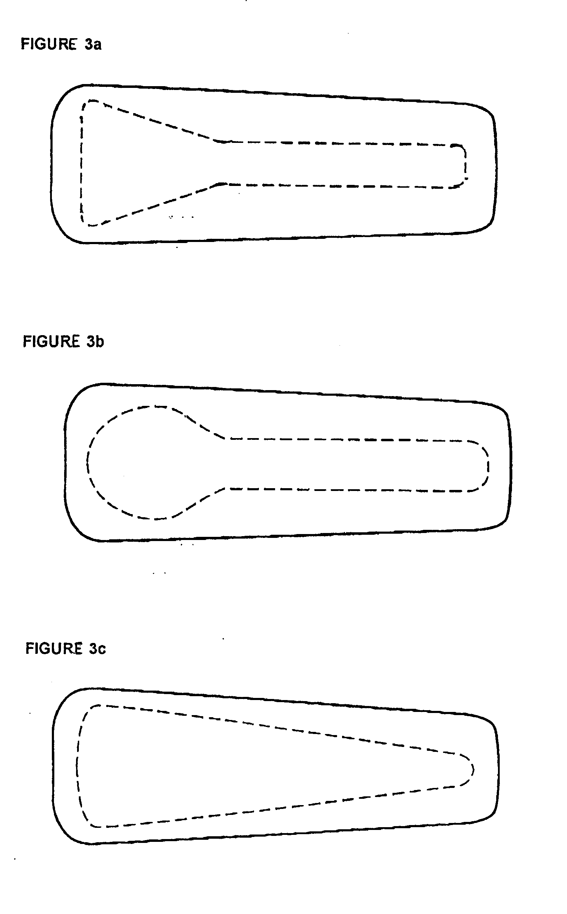

The absorbent core has a suitable shape for the napkin to conform to the tanga-type underwear. In Figure 1 there is shown a triangular shape with slightly concave sides, but it can take any other shape suitable for this use, such as the shapes shown in Figures 3a, 3b or 3c or any other shape (not shown). The absorbent core (16) can comprise fiber-removed cellulose, fiber-removed cellulose combined with superabsorbent material, a compressed material made of fiber-removed cellulose, with or without superabsorbent material, known in industry as "air-laid" material, polyurethane foam or any other material intended for this purpose. The absorbent core has a front section (22) and a back section (24); the front section (22) is the one that will be disposed towards the front of the user during use and is the widest section of the core, while the back section (24) will be disposed during use toward the back of the user and is the narrowest section of the core. The absorbent core (16) also has two longitudinal edges (26) and two transverse edges (28).

Generally, upper layer (12) and lower layer (14) are longer and wider that the absorbent core, forming the contour of the napkin (20); however, the napkin may not have a contour (20), particularly in case the absorbent core (16) is an "air-laid" material or a compressed, fiber removed cellulose material. In this case, the absorbent core (16) and upper layer (12) and lower layer (14) are coterminous, thus avoiding the need to have a contour (20).

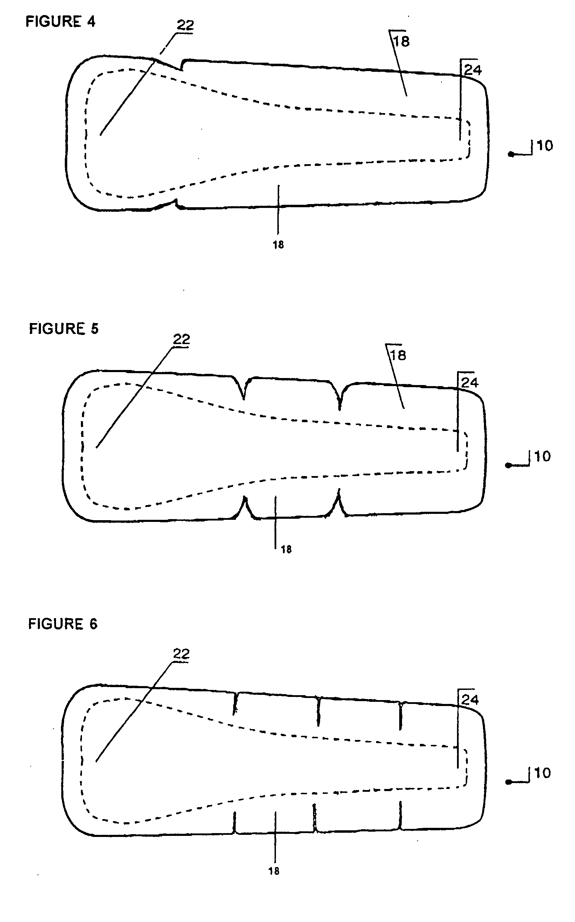

The wings or lateral projections (18) are generally extensions of upper layer (12) and lower layer (14) of the article and extend from the longitudinal edges (26) of the absorbent core (16) or from the contour (20) of the napkin by the lateral sections thereof, and preferably along the entire length of the core; the longitudinal edges (30) of the wings or lateral projections are preferably a straight line, as can be seen in Figure 1, so that same have their narrowest section adjacent the front section (22) of the absorbent core (16) and their widest section "A" adjacent the back section (24) of same. The maximum width "A" that the wings or lateral projections (18) can have is equal to the width of the back section (24) of the absorbent core.
In an alternative embodiment of the invention, the wings or lateral projections do not extended along the entire length of the core, but rather they only span about two third parts of the length thereof, always covering the back section, as can be seen in Figure 4. On the other hand, the wings or lateral projections (18) are not necessarily continuous, rather they can have one or several transverse cuts, such as can be seen in Figures 5 and 6, so to form two or more sections that can be folded over themselves in an independent fashion.

Figure 2 shows an alternative for the wings or lateral projections (16) of the napkin of the present invention. In this case, the widest part "A" of the wings or lateral projections has a dimension equal to half the width of the back section (24) of the absorbent core (16).

Thus, the width "A" of the wings or lateral projections (18) of the napkin of the present invention, in the widest part thereof, may vary between a width equal to half the width of the back section (24) of the absorbent core (16) and a width equal to the width in this back section (24) thereof.

On the other hand, in Figures 3a, 3b and 3c there are illustrated some alternatives of absorbent core (16) designs which are appropriate for use with tanga-style underwear; the napkins also have wings or lateral projections (18) according to the invention.

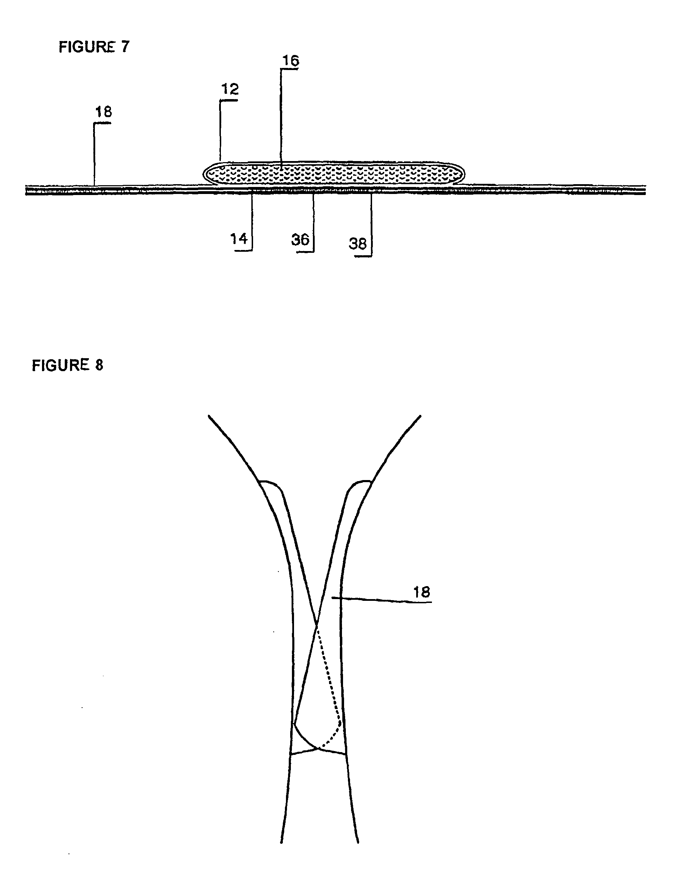

The napkin (10) includes fastening means on the outer face (34) of the lower layer (14) to fasten same to the underwear of the user, as can be seen in Figure 6, which is a section along line A-A' of Figure 1. These fastening means (36) also span the wings or lateral projections (18) and generally comprise one or more strips of pressure sensitive adhesive, which are covered with one or more strips of an easy peelable film (38) whose function is to protect the adhesive to prevent its contamination prior to the use of the napkin. This strip or these strips (38) are removed upon disposing the napkin, keeping the adhesive free for fastening the napkin to the underwear. The wings or lateral projections (18) are folded covering the lateral sections of the tanga-type underwear and sticking to same, thus providing better fastening and protection to the user. Depending on the width of the wings or lateral projections (18), both wings can be fastened to the underwear of the user or, in the case shown in Figure 1, wherein the wings or lateral projections (18) having a width "A" greater that half the back section (24) of the absorbent core (16), one of the wings will be fastened over the other wing, as shown in Figure 7, which illustrates the central section of tanga-type underwear by the outer part thereof, that is, by the part where the wings will be affixed to same.

Several patterns of disposal of pressure sensitive adhesive (36) can be used, such as lines, curves or a continuous layer of adhesive, as shown in Figure 7, so to cover the napkin in its entirety.

On the other hand, this type of articles, although intended for use with tanga-type underwear, can be used with standard underwear, so that the wings or lateral projections (18) do not fold on the edges of the garment, but rather the entire article is affixed to the crotch thereof, providing good protection and maximum comfort, particularly in days of low menstrual flow. This type of article, when used with standard underwear, works very well as a panty-liner.

Although the invention has been described as a function of preferred embodiments thereof, the scope of same includes any change or modification apparent to a person skilled in the art.

## Claims

1. A disposable absorbent article, such as a sanitary napkin (10) or panty-liner comprising a front section (13) and a back section (11) with a design suitable to be used with a tanga-type underwear, comprising an upper layer (12), a lower layer (14) and an absorbent core (16) disposed between them and wings or lateral projections (18); wherein the absorbent core has a front section (22) being the widest section and a back section (24) being the narrowest section **characterized in that**
the wings or lateral projections covering from two third parts of the length of the absorbent core (16) to the entire length of the absorbent core (16) always covering the back section (24) of the absorbent core (16);
the longitudinal edges of the wings or lateral projections are straight lines so that the wings or lateral projections (18) have their narrowest part by the part thereof which is closest to the front section (22) of the absorbent core (16);
wherein the widest part of the wings (18) is that adjacent to the back section (24) of the absorbent core (16); and
wherein the widest part of the wings or lateral projections (18) varies from a dimension equal to half of the width of the back section (24) of the absorbent core (16) to a dimension equal to the width of the back section (24) of the absorbent core (16); and
wherein the wings or lateral projections (18) are divided in two or more sections which can be folded in an independent fashion.

2. A disposable absorbent article according to claim 1, **characterized in that** it includes fastening means (36) to fasten on the underwear of the user, said means spanning the wings or lateral projections (18).

3. A disposable absorbent article according to claim 1, **characterized in that** the wings or lateral projections (18) are an extension of the upper (12) and lower (14) layers of the article.

4. A disposable absorbent article according to claim 3 **characterized in that** the fastening means (36) are a pressure sensitive adhesive.

5. A disposable absorbent article according to claim 4, **characterized in that** the adhesive (36) is applied in continuous lines or in continuous form along the length of the napkin, including the wings or lateral projections (18).

6. A disposable absorbent article according to claim 1, **characterized in that** the article is a sanitary napkin (10).

7. A disposable absorbent article according to claim 1, **characterized in that** the article is a panty-liner.

## Patentansprüche

1. Ein absorbierender Einwegartikel, wie zum Beispiel eine Binde (10) oder Slipeinlage mit einer Vorderseite (13) und einer Rückseite (11) und in einer Ausführung, die für den Gebrauch mit Unterwäsche in der Art eines Tangas passend ist, der eine obere Lage (12), eine untere Lage (14) und eine absorbierende Inneneinlage (16) aufweist, die zwischen den beiden aussenliegenden Lagen angeordnet ist, und mit Flügeln oder seitlichen Fortsätzen (18); Einwegartikel, in dem die absorbierende Inneneinlage einen vorderen Abschnitt (22) aufweist, der den breiteren Teil bildet, und mit einem hinteren Abschnitt (24), der den schmaleren Teil bildet, **dadurch gekennzeichnet, dass**
die Flügel oder seitlichen Fortsätze zwei Drittel der Länge der absorbierenden Inneneinlage (16) bis zur Gesamtlänge der absorbierenden Inneneinlage (16) deckt, d.h. sie decken immer den hinteren Abschnitt (24) der absorbierenden Inneneinlage (16);
die Längskanten der Flügel oder seitlichen Fortsätze geradlinig sind, so dass der schmalste Teil der Flügel oder seitlichen Fortsätze (18) in dem Bereich liegt, der dem vorderen Abschnitt (22) der absorbierenden Innenlage (16) am nächsten liegt;
wo der breiteste Teil der Flügel (18) der ist, der neben dem hinteren Abschnitt (24) der absorbierenden Innenlage (1) zu liegen kommt;
wo die Abmessungen des breitesten Teils der Flügel oder seitlichen Fortsätze (18) variieren, und zwar zwischen ein halb mal die Breite des hinteren Abschnitts (24) der absorbierenden Innenlage (16) und einer Abmessung, die gleich der Breite des hinteren Abschnitts (24) der absorbierenden Innenlage (16) ist; und
wo die Flügel oder seitlichen Fortsätze (18) in zwei oder mehr Abschnitte aufgeteilt sind, die unabhängig voneinander gefaltet werden können.

2. Ein absorbierender Einwegartikel übereinstimmend mit Anspruch 1, **dadurch gekennzeichnet, dass** er ein Befestigungsmittel (36) für die Befestigung an der Unterwäsche des Benutzers aufweist, wobei besagtes Befestigungsmittel die Flügel oder seitlichen Fortsätze (18) spannt.

3. Ein absorbierender Einwegsartikel übereinstimmend mit Anspruch 1, **dadurch gekennzeichnet, dass** die Flügel oder seitlichen Fortsätze (18) eine Verlängerung der oberen Lage (12) und unteren Lage (14) des Artikels sind.

4. Ein absorbierender Einwegsartikel übereinstimmend mit Anspruch 3, **dadurch gekennzeichnet, dass** das Befestigungsmittel (36) ein pressfähiges Haftmittel ist.

5. Ein absorbierender Einwegsartikel übereinstimmend mit Anspruch 4, **dadurch gekennzeichnet, dass** das Haftmittel (36) in fortlaufenden Streifen oder in einer durchgehenden Form über die Länge der Binde, einschliesslich der Flügel oder seitlichen Fortsätze (18) vorgesehen ist.

6. Ein absorbierender Einwegsartikel übereinstimmend mit Anspruch 1, **dadurch gekennzeichnet, dass** der Artikel eine Binde (10) ist.

7. Ein absorbierender Einwegsartikel übereinstimmend mit Anspruch 1, **dadurch gekennzeichnet, dass** der Artikel eine Slipeinlage ist.

## Revendications

1. Article absorbant jetable, comme une serviette hygiénique (10) ou protège-slip comprenant une partie avant (13) et une partie arrière (11) avec une conception qui permet de le porter avec des sous-vêtements de type string, et qui comprend une couche supérieure (12), une couche inférieure (14), un corps absorbant (16) disposé entre elles, ainsi que des ailettes ou rabats latéraux (18) ; le corps absorbant possédant une partie avant (22) qui est la section la plus large et une partie arrière (24) qui est la partie la plus étroite **caractérisé en ce que**
les ailettes ou rabats latéraux, qui couvrent depuis les deux-tiers de la longueur du corps absorbant (16) jusqu'à la longueur tout entière du corps absorbant (16), couvrent toujours la partie arrière (24) du corps absorbant (16) ;
les bords longitudinaux des ailettes ou rabats latéraux sont des lignes droites de sorte que les ailettes ou rabats latéraux (18) aient leur partie plus étroite à l'endroit le plus proche de la partie avant (22) du corps absorbant (16) ;
où la partie la plus large des ailettes (18) est celle adjacente à la partie arrière (24) du corps absorbant (16) ; et
où la partie la plus large des ailettes ou rabats latéraux (18) varie d'une dimension égale à la moitié de la largeur de la partie arrière (24) du corps absorbant (16) à une dimension égale à la largeur de la partie arrière (24) du corps absorbant (16) ; et
où les ailettes ou rabats latéraux (18) sont divisés en deux partie ou plus, qui peuvent être pliées indépendamment.

2. Article absorbant jetable selon la revendication 1, **caractérisé en ce qu'**il inclut des moyens de fixation (36) qui lui permettent d'être attaché au sous-vêtement de l'utilisatrice, lesdits moyens s'étendant sur les ailettes ou rabats latéraux (18).

3. Article absorbant jetable selon la revendication 1, **caractérisé en ce que** les ailettes ou rabats latéraux (18) sont une extension des couches supérieures (12) et inférieures (14) de l'article.

4. Article absorbant jetable selon la revendication 3 **caractérisé en ce que** les moyens de fixation (36) sont un auto-adhésif.

5. Article absorbant jetable selon la revendication 4 **caractérisé en ce que** cet adhésif (36) est appliqué en lignes continues ou avec une forme continue sur la longueur de la serviette, en incluant les ailettes ou rabats latéraux (18).

6. Article absorbant jetable selon la revendication 1 **caractérisé en ce que** l'article est une serviette hygiénique (10).

7. Article absorbant jetable selon la revendication 1 **caractérisé en ce que** l'article est un protège-slip.
